# EUROPÄISCHE PATENTANMELDUNG

(11) **EP 1 502 570 A1**
(43) Veröffentlichungstag der Anmeldung: **02.02.2005**
(21) Anmeldenummer: 03016685.4
(22) Anmeldetag: 01.08.2003
(51) Int. Cl.: A61K 6/083

(54) **Röntgenopakes Dentalmaterial mit oberflächenmodifizierten Nanopartikeln**

(71) Anmelder: Nanosolutions GmbH, 22525 Hamburg (DE)
(72) Erfinder: Ibarra, Fernando, Dr., 20357 Hamburg (DE)
(74) Vertreter: HOFFMANN EITLE

(57) **Zusammenfassung**

Die vorliegende Erfindung betrifft ein Dentalmaterial, das als Füllstoff oberflächenmodifizierte Nanopartikel mit einer mittleren Teilchengröße von weniger als 25 nm enthält, die unter Salzen des Bariums, Strontiums, der Seltenerdmetalle, des Scandiums oder Yttriums ausgewählt werden, deren Oberfläche mit einer organischen Verbindung modifiziert ist, die mit einer N-, P-, S- und/oder O-haltigen funktionellen Gruppe an die Nanopartikel bindet. Dieses Dentalmaterial zeigt nicht nur eine ausgezeichnete Röntgenopazität, sondern auch wichtige verarbeitungstechnische Eigenschaften, wie Transparenz oder Stabilität der Dispersion.

## Beschreibung

Die vorliegende Erfindung betrifft ein röntgenopakes Dentalmaterial, insbesondere ein röntgenopakes Dentalmaterial, das oberflächenmodifizierte Salze der Seltenerdmetalle, des Scandiums, Yttriums, Bariums oder Strontiums enthält, und seine Verwendung in der Dentaltechnik.

### Technischer Hintergrund

Füllungsränder von Zahnfüllungen können vor allem im Seitenzahnbereich oft nur schwer klinisch kontrolliert werden, so dass die radiologische Kontrolle eine wesentliche Unterstützung bei der Diagnose und Differenzialdiagnose von kariösen Läsionen im Füllungsrandbereich, Randspalten und Über- bzw. Unterschuss darstellt. Darüber hinaus kann die röntgenologische Fremdkörperlokalisation auch im Falle versehentlichen Verschluckens oder Aspirierens wichtig sein.

Daher versucht man bereits seit längerer Zeit, Dentalmaterialien so zu modifizieren, dass sie im Röntgenbild einen Kontrast ergeben. Röntgenstrahlen werden von Elementen höherer Ordnungszahl im Periodensystem der Elemente stärker absorbiert als von Elementen niedriger Ordnungszahl. Zur Realisierung eines Kontrasts im Röntgenbild (Röntgenopazität) muss man dem Dentalmaterial daher Elemente mit hohen Atommassen zusetzen. Dies kann prinzipiell über das Monomeroder Füllkörpersystem erfolgen.

So beschreibt die DE 35 02 594 A1 einen röntgenopaken Dentalwerkdstoff auf der Basis von polymersierbaren organischen Bindemitteln, der als Füllstoffe mindestens ein Fluorid der Seltenerdmetalle, vorzugsweise Ytterbiumtrifluorid enthält. Für diese Teilchen wird eine mittlere Primärteilchengröße von 5-700 nm angegeben, obwohl zum Zeitpunkt dieser Anmeldung keine Synthesen für Seltenerdmetalle mit einer Primärteilchengröße von 5-30nm zur Verfügung standen und selbst zu diesem Zeitpunkt bekannte Mahltechniken nicht in der Lage waren, so kleine Teilchen zu erzeugen.

Die Einleitung der DE 35 02 594 A1 verweist auf weitere röntgenopake Füllstoffe. Beispielsweise sind aus der DE-OS 24 58 380 Zahnfüllmassen bekannt, die u.a. Oxide und/oder Carbonate des Lanthans enthalten können.

Bariumsulfat wird als röntgenstrahlenundurchlässiges Material in der DE-OS 24 20 531 und in der EP-A-0 011 735 erwähnt. Die EP-Schrift offenbart ebenfalls lanthanhaltige Röntgenkontrastmittel. DE-OS 29 35 810 schlägt die Oxide der Seltenenerden (Element 57 bis 71) als Röntgenkontrastmittel vor, wobei jedoch offenbar Probleme wegen unerwünschter Verfärbungen auftauchen.

Aus der EP 0 894 488 A2 sind ferner röntgenopake Dentalmassen bekannt, die als Füllstoff Mischkristalle enthalten, in denen die beiden Metallatome des Mischkristalls unter Seltenerdmetallen, Yttrium, Scandium, Zirkonium oder Strontium ausgewählt werden und die Gegenionen Chalkogenide und/oder Halogenide sind. Zu der Partikelgröße der Teilchen wird in dieser Schrift nichts gesagt.

Viele bekannte röntgenopake Füllstoffmaterialien weisen jedoch den Nachteil auf, dass sie nur schwer in der Matrix zu dispergieren sind und/oder keine Anbindungsmöglichkeiten aufweisen. Glasartige Füllstoffe lassen sich beispielsweise mittels der Silanisierung über Hydroxyl- oder Oxygruppen der Glaspartikel besser in die Polymermatrix von Dentalmassen einarbeiten. Fluoridverbindungen sind jedoch nicht silanisierbar und bewirken im ausgehärteten Füllmaterial eine unzureichende physikalische und chemische Resistenz.

Darüber hinaus wird für manche Dentalmaterialien eine möglichst hohe Transparenz gewünscht, die sich mit den bekanntermaßen herstellbaren Teilchengrößen nicht erzielen lässt.

Nachteilig an den bekannten röntgenopaken Füllstoffen war es ferner, dass deren Dispersionsverhalten nicht flexibel an die Art der Matrix (hydrophob bzw. hydrophil) angepasst werden konnte.

Eine Aufgabe der vorliegenden Erfindung war es daher, ein Dentalmaterial mit ausgezeichneter Röntgenopazität bereitzustellen, bei dem sich der röntgenopake Füllstoff gut in der Matrix dispergieren lässt und das ausgezeichnete verarbeitungstechnische Eigenschaften, wie Stabilität und Härtbarkeit zeigt.

Eine weitere Aufgabe der Erfindung beinhaltet Verbesserungen bei der Transparenz des Dentalmaterials.

Schließlich zielt die Erfindung darauf ab, ein Dentalmaterial bereitzustellen, dessen röntgenopaker Füllstoff sich flexibel an die Art der Matrix anpassen lässt.

### Kurze Beschreibung der Erfindung

Diese Aufgaben werden durch ein Dentalmaterial gelöst, das als röntgenopaken Füllstoff oberflächenmodifizierte Nanopartikel mit einer mittleren Teilchengröße von weniger als 25 nm enthält, die unter Salzen des Bariums, Strontiums, der Seltenerdmetalle, des Scandiums oder Yttriums ausgewählt werden, und deren Oberfläche mit einer organischen Verbindung modifiziert ist, die mit einer N-, P-, S-, und/oder O- haltigen funktionellen Gruppe an die Nanopartikel bindet.

Die vorliegende Erfindung betrifft auch verschiedene dentaltechnische Verwendungen, wie sie in den Ansprüchen definiert sind.

### Detaillierte Beschreibung der Erfindung

Das erfindungsgemäße Dentalmaterial enthält als röntgenopaken Füllstoff mindestens ein nanopartikuläres Material, das unter Salzen der Seltenerdmetalle, des Scandiums, des Yttriums, des Bariums und Strontiums ausgewählt wird.

Der röntgenopake Füllstoff ist vorzugsweise schwerlöslich. Der Ausdruck "schwerlöslich" bezieht sich auf die Löslichkeit des röntgenopaken Füllstoffs im menschlichen Speichel. Durch eine geeignete und fachbekannte Auswahl muss somit sichergestellt werden, dass der röntgenopake Füllstoff sich nicht in einem Ausmaß löst, welches die menschliche Gesundheit und/oder die Stabilität der Matrix beeinträchtigt. Als schwerlösliche Salze eigene sich vorzugsweise Sulfate, Phosphate oder Fluoride.

Unter den Salzen der Seltenerdmetalle (Element 57-71), des Scandiums oder des Yttriums sind die Trifluoride bevorzugt.

Zu den bevorzugten Seltenerdmetallen zählen Lanthan, Cer, Samarium, Gadolinium, Dysprosium, Erbium oder Ytterbium. Unter deren Salzen sind die Fluoride bevorzugt, insbesondere Ytterbiumtrifluorid (YbF₃).

Bevorzugte Barium- und Strontiumsalze sind Fluoride, Phosphate und Sulfate, insbesondere die Sulfate.

Die Form des röntgenopaken Füllstoffs unterliegt keinen speziellen Beschränkungen. Diese kann nadelförmig, ellipsoid (eiförmig) oder im wesentlichen kugelförmig sein, wobei die beiden letzten Optionen bevorzugt sind.

Der erfindungsgemäß eingesetzte röntgenopake Füllstoff hat eine mittlere Teilchengröße (Durchmesser), entlang der längsten Achse gemessen, von weniger als 25 nm. Mittlere Teilchengrößen von 1-20 nm, 1-10 nm und 1 bis weniger als 5 nm (z.B. 4,5 nm) sind noch stärker bevorzugt. Die Standardabweichung beträgt in jedem Fall vorzugsweise weniger als 30%, insbesondere weniger als 10%.

Man kann die Teilchengröße und- verteilung nach Verfahren bestimmen, wie sie in der Literatur für Nanopartikel angeben werden, bsp. bei K. Riwotzki et al., J. Phys. Chem. B 2000, 104, 2824-2828, "Liquid phase synthesis of doped nanoparticles: colloides of luminescing LaPO₄:Eu and CePO₄: Tb particles with a narrow particle size distribution", d.h. mit einer transmissionselektronenmikroskopischen (TEM) Untersuchung. Die Gelpermeationschromatographie und Ultrazentrifugation ermöglichen ebenfalls eine Größenbestimmung, wobei TEM- Messungen in punkto Genauigkeit bevorzugt sind.

Die Oberfläche dieser röntgenopaken Nanopartikel wird mit einer organischen Verbindung modifiziert, die mit einer N- ,P-, S- und/oder O- haltigen funktionellen Gruppe an die Nanopartikel bindet.

Diese organische Verbindung hat vorzugsweise von 2-60 Kohlenstoffatome. Die an die Oberfläche der Nanopartikel bindende funktionelle Gruppe kann beispielsweise unter primären oder sekundären Aminen (z.B. Mono-oder Dialkylaminen), Phosphinsäureestern, Phosphonsäurediestern, Phosphorsäuretriestern (z.B. Trialkylphosphaten), Phosphinen (z.B. Trialkylphosphinen), Phosphinoxiden (z.B. Trialkylphosphinoxiden), Sulfoxid (z.B. DMSO), Hydroxy (z.B. organische Polyole), Carbonsäuren oder Carbonsäureestern ausgewählt werden. Je nach Art des Dentalmaterials, in das der röntgenopake Füllstoff eingearbeitet werden soll, d.h. insbesondere je nach Grad der Hydrophilie/Hydrophobie, kann man eine geeignete Zahl und Art funktioneller Gruppen und die Größe des organischen Restes auswählen.

Möchte man das röntgenopake Material in eine hydrophile Matrix (Dentalmaterial) einarbeiten, verwendet man vorzugsweise oberflächenmodifizierende Verbindungen mit kleinerer Kohlenstoffzahl (von 2-10), die ferner vorzugsweise zwei polare funktionelle Gruppen aufweisen, z.B. Polyole. Von diesen funktionellen Gruppen bindet eine an die Oberfläche des Füllstoffes, währende die andere Hydrophilie vermittelt. Zum Einarbeiten in hydrophile Matrizes eignen sich ferner Füllstoffe, die mit einem Sulfoxid, wie DMSO modifiziert wurden.

Möchte man den röntgenopaken Füllstoff in eine hydrophobe Matrix einarbeiten, arbeitet man vorzugsweise mit oberflächenmodifizierenden Verbindungen, die eine Kohlenstoffzahl von 10-60, insbesondere 12-40 aufweisen. Als funktionelle Gruppe eignen sich besonders die bereits erwähnten N- oder P- haltigen funktionellen Gruppen.

Es ist jedoch auch möglich, die oberflächenmodifizierende organische Verbindung mit einer polymerisierbaren Doppelbindung zu versehen, um eine optimale Einbindung in ein polymerisierbares Dentalmaterial oder seine Ausgangsstoffe zu gewährleisten. Hierzu kann man z.B. Verbindungen mit den bereits erwähnten funktionellen Gruppen einsetzen (z.B. primäre oder sekundären Amine, Phosphinsäureester, Phosphonsäurediester, Phosphorsäuretriester, Phosphine, Phosphinoxide, Sulfoxide oder Alkohole, Carbonsäuren und Carbonsäureester, wie sie später näher erläutert werden), in denen zumindest eine Alkylgruppe durch eine Alkenylgruppe, vorzusweise eine mit einer endständigen Doppelbindung ersetzt wurde. Besonders bevorzugt ist die Oberflächenmodifizierung mit Carbonsäuren und Carbonsäureestern mit einer Kohlenstoffzahl von 3 bis 40, stärker bevorzugt 3 bis 30, insbesondere 3 bis 20, die eine polymerisierbare Vinylbindung aufweisen. Besonders bevorzugt ist der Einsatz von Verbindungen mit einer (Meth)acrylatgruppe, z.B. Acrylsäure, Methacrylsäure, Methylacrylat oder Methylmethacrylat und deren Derivaten.

Diese polymerisierbaren Verbindungen lässt man vorzugsweise erst nach Synthese der Nanopartikel mit deren Oberfläche regieren. Zu diesem Zweck wird das Syntheseprodukt in einem Überschuss der polymerisierbaren Verbindung erhitzt, z.B. auf 80 bis 160°C, insbesondere 100 bis 140°C. Weist das Syntheseprodukt bereits an der Oberfläche eine organische Verbindung auf (z.B. das kristallwachstumssteuernde Lösungsmittel aus der Synthesemischung), so kann diese gegen die polymerisierbare oberflächenmodifizierende Verbindung ausgetauscht werden.

Bifunktionelle oberflächenmodifizierende Verbindungen, z.B. Polyole können auch mit einer Verbindung mit einer "reaktiven" Gruppe und einer polymerisierbaren Doppelbindung umgesetzt werden. "Reaktiv" bedeutet hier die Fähigkeit mit der freien (nicht an die Nanopartikeloberfläche bindenden) funktionellen Gruppe eine Bindung einzugehen. Polyole kann man z.B. mit Carbonsäuren mit einer polymerisierbaren Doppelbindung, z.B. (Meth)acrylsäure und ihren Derivaten, unter Bildung einer Esterfunktion umsetzen. Die Reaktion von Polyolen mit Silanen mit mindestens einer reaktiven Alkoxygruppe und einer polymerisierbaren Doppelbindung wie α-Methacryloxypropyltrimethoxysilan führt zur Ausbildung von C-O-Si-Bindungen.

Erfindungsgemäß ist es bevorzugt, den röntgenopaken Füllstoff in einer Synthesemischung herzustellen, welche die oberflächenmodifizierende Verbindung bereits enthält. Es ist jedoch auch möglich, wie bereits geschildert, nach bereits erfolgter Synthese der Nanopartikel die Oberfläche durch Reaktion mit dem oberflächenmodifizierenden Mittel zu behandeln, z.B. durch Austausch der bestehenden Lösungsmittelhülle mit der gewünschten Oberflächenmodifizierenden Verbindung. Dieser "Lösungsmittelaustausch" erfolgt vorzugsweise bei erhöhter Temperatur, typischerweise bei Temperaturen von 50-200 °C.

Im folgenden wird die Synthese erfindungsgemäß geeigneter röntgenopaker Füllstoffe erläutert. Salze, insbesondere Fluoride, Phosphate und Sulfate der Seltenerdmetalle, des Scandiums, Yttriums, Bariums oder Strontiums können so synthetisiert werden, wie dies in der WO 02/20696 beschrieben ist. Dort finden sich z.B. konkrete Beispiele für die Synthese von dotierten Seltenerdmetallfluoriden, die in ihrer Synthesetechnik auf die vorliegende Erfindung übertragbar sind, abgesehen davon das keine Dotierung erforderlich ist.

Die entsprechend Synthese umfasst die folgenden Verfahrensschritte:
a) das Umsetzen, in einem organischen Reaktionsmedium, das mindestens eine oberflächenmodifizierende Verbindung mit einer N- oder P- haltigen funktionellen Gruppe umfasst, und gegebenenfalls mindestens ein weiteres Lösungsmittel, einer im Reaktionsmedium löslichen oder dispergierbaren Metallquelle und einer im Reaktionsmedium löslichen oder dispergierbaren Fluorid-Phosphat- oder Sulfatquelle,
b) gegebenenfalls das Entfernen des Reaktionsmediums von dem gebildeten nanopartikulären Metallfluorid, -phosphat oder -sulfat, und
c) gegebenenfalls die Isolierung des nanopartikulären Salzes.

Der Siedepunkt des organischen Reaktionsmediums liegt vorzugsweise oberhalb der im folgenden angegebenen Reaktionstemperaturen. Der Siedepunkt liegt vorzugsweise bei 150-400 °C, insbesondere im Bereich von 180-300°C (jeweils bei Normaldruck, 1013 mbar). In Abhängigkeit von der Oxidationsempfindlichkeit der Metallquelle, ist es bevorzugt die Reaktion unter einem Inertgas, wie Stickstoff oder Argon durchzuführen.

Vorzugsweise führt man die Reaktion bei einer Temperatur von 50-350°C, z.B. 120-320 °C, insbesondere 180-290°C durch. Eine geeignete Temperatur lässt sich über die Kontrolle der Partikelbildung leicht bestimmen, z.B. indem man die Partikel aus Proben des Reaktionsmediums ausfällt, was durch Abkühlen oder Zugabe eines fällenden Lösungsmittels (z.B. Methanol) bewirkt werden kann und dann das Partikelwachstum untersucht.

Geeignete Reaktionszeiten können auf die gleiche Weise bestimmet werden und liegen vorzugsweise im Bereich von 10min -48h, insbesondere 30min- 20h.

Nach Beendigung der Reaktion, lässt man die Reaktionsmischung typischerweise auf Raumtemperatur abkühlen. Wenn die Nanopartikel dann noch nicht vollständig gefällt sind, kann man durch Methanolzugabe maximale Ausbeuten erzielen.

Die erfindungsgemäß eingesetzte oberflächenmodifizierende organische Verbindung wirkt sich nicht nur positiv auf die spätere Dispergierung in einem Dentalmaterial aus. Sie zeigt zusätzlich eine das Kristallwachstum begrenzende Wirkung und führt zu den zuvor angegebenen geringen Teilchengrößen von weniger als 25nm in enger Teilchengrößenverteilung.

Die oberflächenmodifizierende Verbindung ist vorzugsweise eine phosphororganische Verbindung oder ein mono- oder disubstituiertes Amin. Bevorzugte Ausführungsformen des Amins sind Mono- oder Dialkylamine, in denen der Alkylrest vorzugsweise von 4-20, insbesondere von 6-14 Kohlenstoffatome aufweist, beispielsweise Dodekylamin oder Bis(ethylhexylamin). Die phosphororganischen Verbindungen werden vorzugsweise unter den folgenden Substanzen ausgewählt:
a) Phospinsäureester 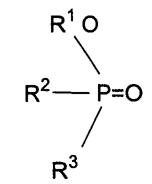
b) Phosphonsäurediester 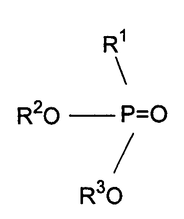
C) Phosphorsäuretriester, insbesondere Trialkylphosphate, wie Tributylphosphat oder Tris(ethylhexyl)phosphat 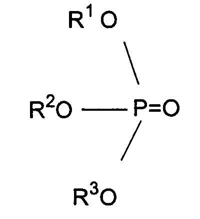
d) Trialkylphosphine, wie Trioktylphospin (TOP) oder 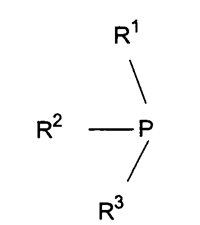
e) Trialkylphosphinoxide, wie Trioktylphosphinoxide (TOPO) 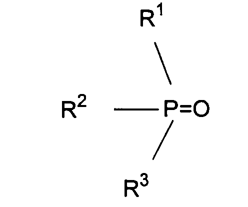
worin R₁, R₂ und R₃ unabhängig voneinander ein verzweigter oder linearer aliphatischer Rest (vorzugsweise Alkyl), ein aliphatisch-aromatischer oder aromatisch-aliphatischer Rest, oder ein aromatischer Rest sind, die jeweils von 4-20, stärker bevorzugt von 4-14, insbesondere von 4-10 Kohlenstoffatome aufweisen. Ein Beispiel für einen aromatischen Rest ist Phenyl, Beispiele für aliphatischaromatische Reste sind Tolyl oder Xylyl, und Benzyl ist ein Beispiel für einen aromatisch-aliphatischen Rest. Unter diesen Phosphororganischen Verbindungen sind (a) bis (c) und (e), insbesondere (a) bis (c) bevorzugt.

Die oberflächenmodifizierende organische Verbindung, d.h. bei der hier beschriebenen Synthese die stickstoff- oder phosphororganische Verbindung kann das einzige Lösungsmittel im Reaktionsmedium sein. Es wird dann vorzugsweise in einer Menge von mindestens 10mol, bezogen auf die molare Menge der als Metallquelle eingesetzten Metallatome verwendet. Eine bevorzugte obere Grenze liegt bei etwa 1000 mol.

Je nach Art des oberflächenmodifizierenden Mittels und insbesondere der Länge des hydrophoben Molekülanteils, kann die Verwendung größerer Mengen der stickstoff- oder phosphororganischen Verbindung im Hinblick auf eine vollständige Fällung der Nanopartikel unerwünscht sein.

Daher ist es bevorzugt, zusätzlich mindestens ein weiteres Lösungsmittel zu verwenden. In dieser Ausführungsform wird die oberflächenmodifizierende organische Verbindung ("erstes Lösungsmittel") vorzugsweise in einer molaren Menge von weniger als 10mol, insbesondere in einer Menge von 0,9-6 mol, bezogen auf 1mol der Metallionen (Metallquelle) eingesetzt. Die Menge des (der) weiteren Lösungsmittel beträgt vorzugsweise von 5-100 mol, bezogen auf 1mol Metallatome (Metallquelle).

Die (das) weitere(n) Lösungsmittel sollten mit der oberflächenmodifzierenden stickstoff- oder phosphororganischen Verbindung mischbar sein. Sie haben vorzugsweise einen Siedepunkt oberhalb von 150°C, insbesondere oberhalb von 180°C. Als bevorzugte Obergrenze kann man 350-400°C angeben.

Das weitere Lösungsmittel kann ein Kohlenwasserstoff sein oder eine organische Verbindung mit mindestens einer polaren Gruppe. Letztere sind bevorzugt, wenn in dem als Ausgangsmaterial verwendeten Metallsalz Kristallwasser vorliegt. Das bzw. die weiteren Lösungsmittel werden vorzugsweise unter den folgenden Verbindungen ausgewählt:
- Lösungsmittel mit mindestens einer Etherfunktion, insbesondere Dialkylether mit 5-10 Kohlenstoffatomen pro Alkylgruppe, wie Dipentylether, Dihexylether, Diheptylether, Dioctylether oder Diisoamylether; Diarylether oder Diaralkylether mit insgesamt 12-18 Kohlenstoffatomen, wie Diphenylether oder Dibenzylether; oder Mono- oder Polyethylenglycol(PEG)dialkylether (worin jede Alkylgruppe vorzugsweise 1-4 Kohlenstoffatome aufweist und die mittlere Zahl der PEG-Einheiten vorzugsweise bis zu 10 beträgt), wie Diethylenglykoldibutylether, Triethylenglykoldibutylether und/oder Tetraethylenglykoldimethylether;
- Verzweigte oder nicht verzweigte Alkane, die vorzugsweise von 10-18 Kohlenstoffatomen, insbesondere von 12-16 Kohlenstoffatomen aufweisen, wie Dodekan oder Hexadecan; und/oder
- Eine organische hochsiedende Base, vorzugsweise eine N-haltige aliphatische Base, am stärksten bevorzugt ein tertiäres substituiertes Amin, insbesondere Trialkylaminverbindungen mit 5-10 Kohlenstoffatomen pro Alkylgruppe, wie Trioktylamin oder Tris(2-ethylhexyl)amin oder eine N- haltige aromatische Base mit vorzugsweise 3-20 Kohlenstoffatomen, wie Imidazol.

Diese Lösungmittel kann man auch in Kombination einsetzen.

Wenn man eine Säure, wie Phosporsäure, Flusssäure (HF) oder Schwefelsäure als Anionenquelle einsetzte, ist es bevorzugt die organische hochsiedende Base in einer etwa äquimolaren Menge (z.B. 0,6-1,4 mol) bezüglich der Wasserstoffatome der Säure einzusetzen.

Die Kationenquelle ist irgendein ausreichend reaktives Metallsalz und vorzugsweise ein Metallchlorid, Metallalkoxid (worin das Alkoxid vorzugsweise von 1-6 Kohlenstoffatome, insbesondere von 1-4 Kohlenstoffatome aufweist), ein Metallnitrat oder Metallacetat. Die Verwendung von Metallchloriden ist besonders bevorzugt. Wenn man hydratisierte Metallsalze einsetzt, ist es bevorzugt das Kristallwasser vor der Reaktion zu entfernen.

Die Anionenquelle für Phosphate, Fluoride oder Sulfate wird vorzugsweise unter den folgenden Verbindungen ausgewählt:
a) Schwefelsäure, Phosphorsäure oder HF,
b) Phosphat-, Sulfat- oder Fluoridsalze, die löslich oder zumindest dispergierbar in der Synthesemischung sind, insbesondere Salze mit einem organischen Kation oder Alkalimetallsalze, oder
C) Ester, die bei höheren Temperaturen Sulfat-, Phophat- oder Fluoridanionen freisetzen, wie Schwefelsäurealkylester.

Das unter Option (b) erwähnte Kation wird vorzugsweise unter basischen N- haltigen aliphatischen, aromatischen, aliphatisch-aromatischen oder aromatisch-aliphatischen Substanzen ausgewählt, die vorzugsweise 4-30, insbesondere 4-20 Kohlenstoffatome aufweisen. Geeignete Kationen umfassen beispielsweise quaternäre Ammonium- und Phosphoniumsalze oder protonierte aromatische Basen, wie Pyridin oder Collidin. Für die Herstellung von Phosphatnanopartikeln kann man beispielsweise Tetrabutylammoniumdihydrogenphosphat, Tetramethylammoniumdihydrogenphophat oder Triethylammoniumdihydrogenphosphat als Anionenquelle einsetzen. Dementsprechend lassen sich Sulfatnanopartikel beispielsweise aus Tetrabutylammoniumhydrogensulfat, Tetramethylammoniumhydrogensulfat, bis-Tetrabutylammoniumsulfat oder Triethylammoniumhydrogensulfat herstellen. Bei der Herstellung von Fluoridnanopartikeln kann man beispielsweise Triethylamin-Trishydrofluorid, Tetrabutylammoniumfluorid, Tetrabutylammoniumhydrogendifluorid, Dodecylaminhydrofluorid oder Pyridinhydrofluorid bzw. Collidinhydrofluorid verwenden.

Löst sich die Kationenquelle zu langsam in dem organischen Synthesemedium, ist es bevorzugt diese zunächst in einem niederen Alkohol, wie Methanol zu lösen, bevor man die oberflächenmodifizierende organische Verbindung und gegebenenfalls weitere Reaktionslösungsmittel zugibt.

Niederer Alkohol und Kristallisationswasser werden dann destillativ entfernt und der Rückstand getrocknet, bevor man weitere Reaktionspartner zugibt.

Die gemäß dieser Synthesetechnik erhaltenen Sulfate, Phosphate oder Fluoride sind sehr gut in herkömmlichen Dentalmaterialien, insbesondere hydrophoben Dentalmaterialien dispergierbar. Hierzu bedarf es keiner weiteren oberflächenmodifizierenden Verfahrenschritte, auch nicht der Silanisierung.

Möchte man die so hergestellten Nanopartikel in hydrophilen Dentalmaterialien dispergieren, kann man die Dispergierbarkeit gegebenenfalls dadurch fördern, dass man die noch aus der Reaktionsmischung stammende oberflächenmodifizierende organische Verbindung durch eine andere oberflächenmodifizierende organische Verbindung mit einer N-, P-, S- und/oder O- haltigen funktionellen Gruppe und einer weiteren polaren Gruppe austauscht. Diese weitere polare Gruppe vermittelt dann die erforderliche Hydrophilie. Alternativ kann man bereits die Synthese der Nanopartikel in einer oberflächenmodifizierenden organischen Verbindung durchführen, welche diese zusätzliche polare Gruppe aufweist, beispielsweise eine Carboxygruppe, Aminogruppe oder Hydroxygruppe. Zu diesem Zweck verwendet man vorzugsweise N-oder P- haltige organische Verbindungen, wie sie zuvor beschrieben wurden, deren organische Reste (z.B. R₁, R₂, R₃) eine weitere polare Gruppe, z.B. Carboxyl, Amino- und/oder Hydroxy aufweisen. Diese polare Gruppe befindet sich vorzugsweise an dem der Partikeloberfläche gegenüberliegenden Ende des Moleküls.

Diese doppelte Funktionalisierung der Nanopartikel kann auch dann von Interesse sein, wenn man weitere oberflächenmodifizierende Schritte, z.B. eine Silanisierung durchführen möchte, um die Nanopartikel in speziellen Dentalmaterialien optimal zu dispergieren.

Als nächstes wird nun die Synthese von nanopartikulären Barium- und Strontiumsulfaten mit einem Polyol oder Sulfoxid als Lösungsmittel und oberflächenmodifizierende Verbindung beschrieben.

Die hierfür geeigneten Polyole haben vorzugsweise 2-3 Hydroxygruppen. Beispiele hierfür sind Glycerin, Ethylenglycol oder Polyethylenglycol bei einer bevorzugten mittleren Anzahl der Ethylenglycoleinheiten von bis zu 4. Als Sulfoxid kann man Dimethylsulfoxid (DMSO) einsetzen.

Bevorzugte Metallatomquellen sind Bariumchlorid, Strontiumchlorid und ihre Hydrate. Als Ausgangsmaterial für das Sulfatanion verwendet man vorzugsweise Alkalimetallsulfate, Ammoniumsulfat oder Sulfate mit einem organischen Kation. Die entsprechende Hydrogensulfate sind gleichermaßen geeignet.

Das organische Kation wählt man vorzugsweise unter basischen N-haltigen aliphatischen, aromatischen, aliphatisch-aromatischen, und aromatisch-aliphatischen Verbindungen aus, die vorzugsweise von 4-30, insbesondere 4-20 Kohlenstoffatome aufweisen. Geeignete Kationen beinhalten beispielsweise quaternäres Ammonium oder Phosphonium, mit 4 Substituenten, die unabhängig voneinander unter Alkyl mit vorzugsweise 1-10 Kohlenstoffatomen (vorzugsweise 1-5) oder Benzyl ausgewählt werden können, oder protonierte Basen, wie Hydrazin, Amantadin, Pyridin oder Collidin.

Dementsprechend kann man Barium- oder Strontiumsulfatnanopartikel aus Ausgangsstoffen, wie Tetrabutylammoniumhydrogensulfat, Tetramethylammoniumsulfat, Bistetrabutylammoniumsulfat oder Triethylammoniumhydrogensulfat herstellen. Andere geeignete Ausgangsstoffe sind Ammoniumhydrogensulfat, Ammoniumsulfat, Alkalimetallhydrogensulfate, Amantadinsulfat, Ethylendiammoniumsulfat und Hydraziniumsulfat.

Beim Einsatz von Hydrogensulfaten gibt man vorzugsweise organische Basen, wie Imidazol als Säurefänger zu dem Reaktionsmedium.

Die Reaktion wird vorzugsweise bei Temperaturen von 50-240°C durchgeführt, wobei der Temperaturbereich von 50-100°C für Glycerin und höhere Temperaturen im Bereich von 160-240°C, insbesondere 160-180°C für Polyole mit höherem Molekulargewicht oder Sulfoxidlösungsmittel bevorzugt sind.

Die gemäß dieser Synthese erhaltenen Barium- oder Strontiumsulfatnanopartikel sind insbesondere in hydrophilen Dentalmaterialien ausgezeichnet dispergierbar. Möchte man sie in hydrophobe Dentalmaterialien einarbeiten, ist es gegebenenfalls erforderlich, die Barium- oder Strontiumsulfatnanopartikel einer Nachbehandlung zur Oberflächenveränderung zu unterziehen. Beispielsweise kann man das Polyol oder Sulfoxid gegen ein stärker hydrophobes koordinierendes Lösungsmittel austauschen, beispielsweise die zuvor beschriebenen stickstoff- oder phosphororganischen Verbindungen. Die entsprechende Nachbehandlung beinhaltet das Erwärmen der Barium- oder Strontiumsulfatnanopartikel in diesem stärker hydrophoben Lösungsmittel. Die in einem Polyol hergestellten Barium- oder Strontiumsulfate kann man ferner silanisieren, um die Hydrophobie an der Oberfläche zu erhöhen. Zur Silanisierung kann man beispielsweise eine Silanverbindung mit mindestens einer reaktiven Alkoxygruppe und einer polymerisierbaren Doppelbindung wie α-Methacryloxypropyltrimethoxysilan einsetzen. Aus solchen Teilchen kann man mit mindestens einer Vinylverbindung Vorpolymerisate herstellen, aus denen sich eine Matrix mit besonders guter Einbindung der röntgenopaken Füllstoffe erzeugen lässt.

Die so hergestellten Fluoride, Barium- oder Strontiumsalze zeichnen sich durch verschiedene Vorteile gegenüber herkömmlichen röntgenopaken Füllstoffen aus. Sie lassen sich ohne weitere Verfahrensschritte zur Oberflächenmodifizierung sehr gut in Dentalmaterialien dispergieren. Bei einer Teilchengröße von weniger als 25nm findet keine Wechselwirkung mit sichtbarem Licht statt, so dass man erfindungsgemäß transparente Dentalmaterialien herstellen kann. Überraschenderweise zeigen die erfindungsgemäßen Dentalmaterialien auch eine höhere Röntgenopazität gegenüber bekannten Füllstoffen der gleichen chemischen Zusammensetzung, die jedoch eine größere Teilchengröße aufweisen. Ein entscheidender Vorteil der erfindungsgemäßen Teilchen besteht ferner darin, dass sie eine flexible Anpassung an die chemische Beschaffenheit der Matrix ermöglichen.

Das erfindungsgemäße Dentalmaterial umfasst vorzugsweise eine härtbare oder bereits ausgehärtete Matrix. Metalle und Metalllegierungen, wie sie in der Dentaltechnik ebenfalls häufig eingesetzt werden, werden nicht als härtbare oder bereits ausgehärtete Systeme angesehen.

Das Dentalmaterial ist vorzugsweise ein Composit, Compomer, ein Zement oder ein organisches Polymer (synthetisches Harz) bzw. die entsprechende noch nicht gehärtete Monomer- oder Oligomermischung.

Die Zemente können unter fachbekannten Zementen ausgewählt werden, beispielsweise Phosphatzementen, Silicatzementen, Silicophosphatzementen, Zinkoxid-Eugenolzementen, Carboxylatzementen, Glasionomerzementen, Calciumhydroxidzementen, wie sie in Ullmann's Encyclopedia of Industrial Chemistry, Vol. 8, 1987, Seite 274ff beschrieben sind. Als Dentalmaterial auf Polymerbasis kann man die ebenfalls fachbekannten (siehe Ullmann, Seite 277ff) wärmehärtenden, in der Kälte härtenden oder lichthärtenden Polymersysteme einsetzen.

Die Glasionomerzemente wurden in den 60er Jahren von Wilson & Kent eingeführt (Wilson, A.D.; J. Dent. Res. 75 (10), 1723 (1996). Die Dentalmaterialgruppe der Compomere wird beispielsweise in der Patentschrift EP 0 219 058 beschrieben. Carboxylatzemente sind bei D.C. Smith; British Dental J 125, 381 (1968) beschrieben.

Es ist besonders bevorzugt, dass das erfindungsgemäße Dentalmaterial mindestens eine polymerisierbare Vinylverbindung (beziehungsweise ein daraus abgeleitetes Oligomer oder Polymer) enthält. Insbesondere eignen sich hierfür monofunktionelle oder polyfunktionelle Methacrylate, die allein oder in Mischungen eingesetzt werden können. Als Beispiele für diese Verbindungen kommen Methylmethacrylat, Isobutylmethacrylat, Cyclohexylmethacrylat, Triethylenglycoldimethacrylat, Diethylenglycoldimethacrylat, Ethylenglycoldimethacrylat (EGDMA), Polyethylenglycoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, aber auch Bis-GMA sowie die Reaktionsprodukte aus Isocyanaten, insbesondere Di- und/oder Triisocyanaten und OH-gruppenhaltigen Methacrylaten in Frage. Beispiele dafür sind die Umsetzungsprodukte von 1 Mol Hexamethyldiisocyanat mit 2 Mol 2-Hydroxyethylenmethacrylat, von 1 Mol Tri(6-isocyanatohexyl)biuret mit 3 Mol Hydroxyethylmethacrylate und von 1 Mol Trimethylhexamethylendiisocyanat mit 2 Mol Hydroxyethylmethacrylat, die im folgenden als Urethandimethacrylat (UDMA) bezeichnet werden. Der Anteil dieser meist langkettigen Verbindungen im Dentalwerkstoff bewegt sich zwischen 10 und 50 Gew.%. Im Prinzip kommen alle für einen Dentalwerkstoff brauchbaren Bindemittel in Frage.

Bevorzugte fachbekannte Mischungen aus Acrylatmonomeren sind beispielsweise Mischungen aus Urethandimethacrylaten und Polymethylendimethacrylaten, oder Mischungen aus Urethandimethacrylaten und (Poly)-Ethylenglycoldimethacrylaten.

Besonders bevorzugt sind Compositmaterialien, die seit 1962 als Zahnrestaurationsmaterialien bekannt sind (Bowen, R.L.; US 3,066,112), insbesondere die Verwendung des sogenannten Bowen-Monomers Bis-GMA, das aus Bisphenol A-Di(2,3-Epoxypropyl)ether und Acrylsäure hergestellt wird in Kombination mit mindestens einem weiteren Acrylatmonomer.

Beispielsweise kann man polymerisierbare Mischungen aus Bis-GMA und (Poly)-Ethylenglycoldimethacrylaten einsetzen.

Letztere können auch als Zweikomponenten-Zahnfüllungsmaterial eingesetzt werden. Zu diesem Zweck mischt man der sog. "Peroxidpaste" und "Aminpaste" die Monomere, den röntgenopaken Füllstoff, gegebenenfalls einen weiteren Füllstoff, wie amorphes SiO₂, und zur Viskositätseinstellung gegebenenfalls ein Vorpolymerisat aus diesen vier Komponenten und gegebenenfalls Farbpigmente und Stabilisatoren bei, wobei die Peroxidpaste z.B. Benzylperoxid und die Aminpaste ein Amin, wie N,N-Diethanol-p-toluidin oder N,N-Dimethyl-ptoluidin enthält.

Der erfindungsgemäße Dentalwerkstoff enthält vorzugsweise 1-50 Gew.-% der röntgenopaken Nanopartikel, bezogen auf das Gesamtgewicht des Dentalmaterials. Diese können auf die gleiche Weise in das Dentalmaterial eingearbeitet werden, wie dies im Stand der Technik für größere Partikel beschrieben ist.

Das Dentalmaterial kann ferner gegebenenfalls weitere Füllstoffe enthalten. Ein geeigneter Füllstoff ist pyrogene Kieselsäure mit einer BET-Oberfläche von etwas 20-400m²/g und einer mittleren Primärteilchengröße von etwa 5-50nm.

Außerdem können Quarz-, Glaskeramik-, Glaspulver oder Mischungen davon als weiterer Füllstoff eingesetzt werden, z.B. Glaspulver, insbesondere Barium und/oder Strontiumglaspulver, was die Röngenopazität zusätzlich erhöht. Die mittlere Partikelgröße dieser Pulver liegt vorzugsweise im Bereich von 0,4 bis 1,5 µm und insbesondere im Bereich von 0,7 bis 1,0 µm.

Zur Erhöhung der Röngenopazität kann man ferner gefällte Mischoxide, wie beispielsweise ZrO₂/SiO₂ als Füllstoffe eingesetzen. Bevorzugt sind Mischoxide mit einer Partikelgröße von 200 bis 300nm und insbesondere etwa 200 nm, bei einem Brechungsindex von 1,52 bis 1,55. Die Mischoxide sind vorzugsweise kugelförmig und weisen eine einheitliche Größe auf.

Die Füllstoffe für eine organische Matrix sind vorzugsweise zur Verbesserung der Haftung zwischen Füllstoff und Matrix silanisiert.

Bezogen auf das Gesamtgewicht ist der weitere Füllstoff, z.B. die Kieselsäure, das Quarz-, Glaskeramik-, Glaspulver oder das Mischoxid in einer Menge von etwa 5-84 Gew.-% enthalten. Das Gesamtgewicht der anorganischen Füllstoffe im erfindungsgemäßem Dentalmaterial, einschließlich des erfindungsgemäßen röntgenopaken Füllstoffs mit einer Teilchengröße kleiner 25nm, beträgt vorzugsweise 6-85 Gew.-%, insbesondere 45-75 Gew.-%.

Der Dentalwerkstoff kann je nach Art des verwendeten Katalysators heiß, kalt oder durch Photopolymerisation aushärtbar sein.

Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis-(isobutyroethylester), Benzpinakol und 2,2'-Dimethylbenzpinakol sind geeignet.

Als Katalysatoren für die Photopolymerisation können z.B. Benzophenon und seine Derivate sowie Benzoin und seine Derivate verwendet werden. Beispiele für bevorzugte Photosensibilisatoren sind die α-Diketone wie 9,10-Phenathrenchinon, Diacetyl, Furil, Anisil, 4,4'-Dichlorbenzil und 4,4'-Dialkoxybenzil. Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, n-Butylamin, Triethylamin, Triethanolamin, N,N-Dimethylanilin, N-Methyldiphenylamin und N,N-Dimethyl-sym.-xylidin.

Als Katalysatoren für die Kaltpolymersiation werden Radikale liefernde System, z.B. Benzoyl-bzw. Lauroylperoxid zusammen mit Aminen wie N,N-Dimethyl-sym.-xylidin oder N,N-Dimethyl-p-Toluidin verwendet.

Die Menge dieser Katalysatoren im Dentalwerkstoff liegt üblicherweise zwischen 0,1 bis 5 Gew.%

Dem Dentalwerkstoff können ferner feinteilige Splitter- oder Perlpolymerisate einverleibt werden, die Homo- oder Copolymere der schon beschriebenen Vinylverbindungen sein können. Diese Homo-bzw. Copolymeren können ihrerseits mit den hier beschriebenen anorganischen Füllstoffen, auch den röntgenopaken, gefüllt sein. Es wird dazu auf die EP-PS 11 190 und DE-PS 24 03 211 verwiesen. Ferner kann der Dentalwerkstoff die üblichen Pigmentierungsmittel und Stabilisatoren enthalten.

Beispiele für erfindungsgemässe Dentalmaterialien auf Polymerbasis umfassen oder bestehen aus
(a) 10 bis 80 Gew.%, z.B. 10 bis 30 Gew.-% organisches Bindemittel auf Polymerbasis,
(b) ggf. 0,01 bis 5 Gew.-% Additive, wie Stabilisatoren, Polymerisationsinitiatoren, Farbstoffe, Pigmente, etc. und
(c) 6-85 Gew.-%, insbesondere 45-75 Gew.-% Füllstoffe, einschließlich des erfindungsgemäßen röntgenopaken Füllstoffs mit einer Teilchengröße kleiner 25nm.

Vorzugsweise dient der erfindungsgemäße Dentalwerkstoff als Zahnfüllungsmaterial. Dabei soll der Zahnarzt beim Anfertigen von Röntgenbildern in die Lage versetzt werden, aufgrund der Röntgenopazität der Füllung festzustellen, ob sich Sekundärkaries gebildet hat oder nicht. Ein lichthärtendes, röntgenopakes Füllungsmaterial enthält z.B. als Bindemittel ein Urethandimethacrylat bzw. Bis-GMA, Triethylenglycoldimethacrylat als verdünnendes Monomer, röntgenopakes Fluorid, z.B. Ytterbiumtrifluorid oder Barium/Strontiumsalz, einen weiteren Füllstoff, z.B. Quarz-, Glaskeramik-, Glaspulver oder pyrogene Kieselsäure mit einer mittleren Größe der Primärteilchen von 40 nm und einer BET-Oberfläche von 50 m²/g, Campherchinon und N,N-Dimethyl-sym.xylidin als Katalysator sowie Stabilisatoren und Farbpigmente.

Um den Füllungsgrad solcher Füllungsmaterialien zu erhöhen, ist es üblich, z.B aus Bis-GMA, Triethylenglycoldimethacrylat, röntgenopakem Fluorid, z.B. Ytterbiumtrifluorid oder Barium/Strontiumsalz, und der pyrogenen Kieselsäure ein Copolymer herzustellen, dieses als Splitterpolymerisat zu vermahlen und dann in das Füllungsmaterial einzuarbeiten. Die Polymerisation nach dem Legen der Füllung geschieht mit einer handelsüblichen Halogenlampe.

Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste ein Peroxid, z.B. Benzoylperoxid und in die andere Paste ein Amin, z.B. N,N-Dimethyl-p-toluidin eingemischt. Durch Vermischen etwa gleicher Teile der beiden Pasten erhält man ein Zahnfüllungsmaterial, welches in wenigen Minuten aushärtet.

Wenn man bei den letztgenannten Materialien des Amin weglässt und als Katalysator z.B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird von der Kavität im Munde ein Abdruck genommen und ein Gipsmodell hergestellt. In die Kavität des Giopsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Die Erfindung bezieht sich nicht nur auf den röntgenopaken Dentalwerkstoff, sondern auch auf daraus hergestellte Fertigteile, z.B. künstliche Zähne, Schalen, Inlays etc. Sie wird anhand der nachfolgenden Beispiele erläutert.

### Bezugsbeispiel 1: Synthese von BaSO₄

In einem Rundkolben werden 55g BaCl₂ eingewogen und in 300 ml Glycerin gelöst. Anschließend werden 30g in Glycerin gelöstem Imidazol zur Ba-haltigen Lösung gegeben und das Reaktionsgemisch anschließend unter leichtem Erwärmen 24 h getrocknet. Parallel werden 73g Tetrabutylammoniumhydrogensulfat in Glycerin gelöst und über Nacht getrocknet. Beide Lösungen werden dann bei 70 °C zusammengegeben und eine Stunde lang gerührt. Nach Zugabe von 0,5 Äquivalenten Wasser, bezogen auf Glyzerin, werden die somit synthetisierten Bariumsulfatteilchen mit Isopropanol gefällt mit Ethanol gewaschen und anschließend getrocknet. Es ergeben sich ca. 60 g Bariumsulfatnanopartikel mit homogener Grössenverteilung (Standardabweichung 22 %) bei einer mittleren Teilchengröße von 19 nm.

### Bezugsbeispiel 2: Synthese von YbF₃

17,48 g (45 mmol) YbCl₃ x 6 H₂O werden in 17,5 ml MeOH gelöst und mit 125 ml TEHP (Tris-(2-ethylhexyl)phosphat) versetzt. In einem zweiten Gefäß werden 41,17 g (130,5 mmol) Tetrabutylammoniumfluorid x 3 H₂O in 20 ml MeOH gelöst und in 175 ml TEHP aufgenommen. Beide Reaktionslösungen werden bei Raumtemperatur im Vakuum vom Methanol befreit und vereinigt. Das Reaktionsgemisch wird für 2 h auf 200 °C unter Rückfluß erhitzt, der entstehende Niederschlag nach dem Abkühlen zentrifugiert, zweimal mit Methanol gewaschen und getrocknet (Ausbeute 10,08 g, 100,7 % d. Th.).

Röntgendiffraktometrie (XRD)-Messungen der so erhaltenen Teilchen deuteten auf eine mittlere Teilchengrösse von weniger als 10nm.

### Beispiel 1

Ersetzt man in einem bekannte härtbaren Dentalmaterial auf Acrylatbasis das nicht oberflächenbehandelte Ytterbiumfluorid größerer Teilchengröße (z.B. 300 mm) durch das erfindungsgemäß in Bezugsbeispiel 2 hergestellte Ytterbiumfluorid, dessen Oberfläche mit TEHP modifiziert wurde und das eine Teilchengröße von weniger als 10nm zeigt, stellt man nicht nur eine excellente Röntgenopazität fest, sondern überraschenderweise auch Verbesserungen hinsichtlich der Dispergierbarkeit in der Matrix, der Stabilität der Polymermatrix und deren Transparenz. Beispiele für solche herkömmlichen röntgenopaken Dentalmaterialien mit Ytterbiumfluorid als röntgenopaken Füllstoff sind die Beispiele 1, 2 und 3 der DE 35 025 94 A1.

Die Messung der Röntgenopazität erfolgt bezüglich Aluminium gemäß europäischen oder internationalen Normen (EN 24049: 1993 oder ISO 9917).

Darüber hinaus wurde festgestellt, dass die erfindungsgemäßen Dentalmaterialien auch hinsichtlich anderer wichtiger Materialeigenschaften, wie Abrasionverhalten und Polierbarkeit auf Hochglanz gute Eigenschaften zeigen.

### Beispiel 2: Einarbeiten in eine Monomermischung

(Monomermischung: 43 % Nupol, 37 % RM3, 20 % SR205; Nupol: Bisphenol A-diglycidyletherdimethacrylat; RM 3: 7,7,9-Trimethyl-4,13-dioxo-3,14-dioxa-5,12-diazohexadecan-1,16-dimethacrylat, SR 205: Triethylenglykoldimethacrylat)

1 g YbF₃, das wie in Beispiel 2 hergestellt wurde, wird in ca. 20 ml Methylmethacrylat im Mörser verrieben, das Gemisch in einen Rundkolben überführt und auf 120 ° C erhitzt (30 min). Nach Abkühlen auf Raumtemperatur wird evakuiert bis zur Trockne. Das erhaltene Pulver wird mit 10 g der Monomermischung im Mörser vermischt und für mehrere Stunden im Ultraschallbad behandelt. Man erhält eine flüssige, homogene Monomermischung, die nun auf herkömmliche Weise, z.B. durch Zusatz bekannter nichtröntgenopaker Füllstoffe, weiterer Additive, etc. zu einem Dentalmaterial verarbeitetet werden kann.

## Patentansprüche

1. Dentalmaterial, das mindestens einen Füllstoff enthält, der unter Salzen des Bariums, Strontiums, der Seltenerdmetalle, des Scandiums oder Yttriums ausgewählt wird,
**dadurch gekennzeichnet, dass** der Füllstoff in Form oberflächenmodifizierter Nanopartikel mit einer mittleren Teilchengröße von weniger als 25 nm vorliegt, deren Oberfläche mit einer organischen Verbindung modifiziert ist, die mit einer N-, P-, S- und/oder O-haltigen funktionellen Gruppe an die Nanopartikel bindet.

2. Dentalmaterial gemäß Anspruch 1, worin die Nanopartikel unter Fluoriden der Seltenerdmetalle, des Scandiums oder Yttriums, Bariumsulfat und Strontiumsulfat ausgewählt werden.

3. Dentalmaterial gemäß einem der vorangehenden Ansprüche, worin die oberflächenmodifizierende organische Substanz unter Polyolen, Sulfoxiden, Phosphinsäureestern, Phosphonsäurediestern, Phosphorsäuretriestern, Trialkylphosphinen, Trialkylphophinoxiden, Mono- und Dialkylaminen, Carbonsäuren und Carbonsäureestern ausgewählt wird.

4. Dentalmaterial gemäß einem der vorangehenden Ansprüche, worin die oberflächenmodifizierende organische Verbindung eine polymerisierbare Doppelbindung aufweist.

5. Dentalmaterial gemäß einem der vorangehenden Ansprüche, worin die Nanopartikel in einer Menge von 1 bis 50 Gew.%, bezogen auf das Gesamtgewicht des Dentalmaterials vorliegen.

6. Dentalmaterial gemäß einem der vorangehenden Ansprüche, worin der Dentalwerkstoff mindestens eine polymerisierbare Vinylverbindung und ggf. einen weiteren anorganischen Füllstoff umfasst.

7. Dentalmaterial gemäß einem der vorangehenden Ansprüche, worin die Gesamtmenge der anorganischen Füllstoffe 6 bis 85 Gew.% beträgt.

8. Verwendung eines Dentalwerkstoffs gemäß einem der Ansprüche 1 bis 7 als 1- oder 2-Komponenten-Zahnfüllungsmaterial oder Dentalzement.

9. Verwendung eines Dentalwerkstoffs gemäß einem der Ansprüche 1 bis 7 als Kronen- und Brückenmaterial oder Zahnprothesenmaterial.

10. Verwendung eines Dentalwerkstoffs gemäß einem der Ansprüche 1 bis 7 zur Herstellung von künstlichen Zähnen, Inlays, Implantaten und Zahnfertigteilen.
